# EUROPÄISCHE PATENTSCHRIFT

(11) **EP 0 978 259 B1**
(45) Veröffentlichungstag und Bekanntmachung des Hinweises auf die Patenterteilung: **20.02.2002**
(21) Anmeldenummer: 99122177.1
(22) Anmeldetag: 12.08.1992
(51) Int. Cl.: A61B 18/12

(54) **Hochfrequenzchirurgiegenerator mit einer einstellbaren Ausgangsleistung**
Surgical highfrequency generator with selectable power output
Generateur electrochirurgical avec une puissance de sortie selectionnable

(30) Priorität: 12.08.1991 DE 4126609; 24.10.1991 DE 4135185
(43) Veröffentlichungstag der Anmeldung: 09.02.2000
(62) Teilanmeldung aus: 92916904.3
(73) Patentinhaber: Karl Storz GmbH & Co. KG, 78532 Tuttlingen (DE)
(72) Erfinder: Lindenmeier, Heinz Prof. Dr.-Ing., 82152 Planegg (DE); Fastenmeier, Karl Prof. Dr.-Ing., 81739 München (DE); Lohr, Georg Dr., 82223 EICHENAU (DE); Flachenekker, Gerhard Prof. Dr.-Ing. (verstorben), , (DE)
(74) Vertreter: Münich, Wilhelm, Dr.

(56) Entgegenhaltungen:
- DE-A- 3 530 335
- DE-A- 3 622 337

## Beschreibung

### Technisches Gebiet

Die Erfindung bezieht sich auf einen Hochfrequenzgenerator mit einer einstellbaren Ausgangsleistung für miteinander gekoppelte Betriebsarten Koagulieren und nachfolgend Schneiden zur Entfernung der Sonde.

### Stand der Technik

Hochfrequenzströme werden in der Chirurgie zum Abtragen von Gewebeteilen verwendet, wenn der Operationsort durch natürliche Körperöffnungen erreichbar ist, ein Skalpell aber nicht ohne Eröffnung des Körpers des Patienten angesetzt werden kann. Zum Beispiel können in der Urologie mit transurethral eingeführten Operationsinstrumenten und mit Hilfe von Hochfrequenzströmen Tumore aus der Blase abgetragen oder krankhafte Wucherungen der Prostata entfernt werden. In der Enterologie können auf ähnliche Weise z.B. Polypen von der Darmwand abgetrennt werden. Die Schneidelektrode des Operationsinstrumentes hat dabei nur solange eine Schneidwirkung, wie der den Hochfrequenzstrom liefernde Hochfrequenzgenerator aktiviert ist. Damit ist ein gefahrloses Einbringen und Entfemen des Operationsinstrumentes durch die natürlichen Körperöffnungen gewährleistet.

Weiterhin werden Hochfrequenzströme in der Chirurgie zum blutarmen Schneiden und zum Stillen von Blutungen verwendet. Es sind Hochfrequenzgeneratoren bekannt, die sowohl einen sogenannten "Schneidmodus" als auch einen "Koagulationsmodus" aufweisen. Diese Generatoren sind zum Gewebetrennen und zum gezielten Blutstillen, dem Koagulieren geeignet. Sie werden hauptsächlich bei endoskopischen Operationen wie z.B. in der Urologie, der Gynäkologie, der Polypektomie usw. angewandt. Daneben gibt es Hochfrequenzgeneratoren, die nur einen Koagulationsmodus besitzen. Diese sog. Koagulatoren werden in der offenen Chirurgie verwendet, um angeschnittene, stark blutende Gefäße zu verschließen oder großflächige, diffuse Blutungen zu stillen.

Ein Problem in der Hochfrequenzchirurgie ist die richtige Dosierung der momentan applizierten Hochfrequenzleistung. Die für gute Schneidwirkung mindestens notwendige Hochfrequenzleistung kann sehr stark schwanken. Sie hängt von der Gewebebeschaffenheit, der Leitfähigkeit und dem Wassergehalt des Gewebes, der Elektrodenform und Elektrodengröße, der Schnitt-Tiefe und Schnittgeschwindigkeit und weiteren elektrischen Parametern ab, die alle im Laufe einer Operation gewissen, oft sehr abrupt auftretenden Änderungen unterworfen sind. Die übliche, aus der Erfahrung des Operateurs gewonnene Einstellung des Hochfrequenzgenerators führt daher im Mittel zu einer deutlich überhöhten Hochfrequenzleistung, deren Gefahren der Operateur seinen Patienten und sich selbst bewusst aussetzen muss. Um diese Gefahren so klein wie möglich halten zu können bzw. nahezu vollständig ausschließen zu können, müsste die momentane Ausgangsleistung des Hochfrequenzgenerators automatisch so geregelt sein, dass sie in jedem Zeitmoment dem absolut notwendigen Mindestmaß entspricht.
Eine Erhöhung der zugeführten Leistung hat eine höhere Koagulation des Gewebes an der Schnittfläche zur Folge. Damit lässt sich eine Blutstillung erreichen. Diese ist bei Operationen in Geweben mit ohnehin geringer Blutungsneigung nicht unbedingt notwendig. Auch bei Operationstechniken, bei denen große Gewebevolumen abgetragen werden wie z.B. in der Urologie ist eine
Schnittflächenkoagulation nicht unbedingt bereits während des Schneidens nötig. Denn hier wird mit den nachfolgenden Schnitten weiter in tieferliegende Gewebeschichten geschnitten. Eine geringe, definierte Koagulation ist jedoch häufig erwünscht um überflüssige Blutungen zu vermeiden und das Operationsfeld übersichtlich zu halten. Bei anderen Operationsarten, wie bei der Polypektomie, bei denen die ganze Operation im Optimalfall aus einem einzigen Schnitt besteht, ist jedoch bereits während des Schnittes eine optimale Koagulation unabdingbar.

Beim Schneiden mit Hochfrequenzströmen wird ein kontinuierlicher Hochfrequenzstrom verwendet. Bei der Hochfrequenzkoagulation wird die Joule'sche Wärme des Hochfrequenzstromes zur Stillung von Blutungen verwendet. Dazu wird ein Hochfrequenzstrom von einer Koagulationssonde auf das Gewebestück übergeleitet, auf dessen Oberfläche sich die Blutung befindet. Die Blutung kann von einem angeschnittenen Gefäß - meistens einer Arterie - oder großflächig in Form einer diffusen Blutung von vielen kleinen aufgetrennten Mikrogefäßen ausgehen. Bei der Koagulation kann man zwischen zwei Verfahren unterscheiden. Die Niederspannungskoagulation verwendet meist einen kontinuierlichen Hochfrequenzstrom, der so niedrig gewählt ist, dass ein Schneideffekt der Sonde nicht auftreten kann. Zur Hochspannungskoagulation dagegen verwendet man ausschließlich gepulste Hochfrequenzströme. Hier ist die mittlere Leistungszufuhr so gering, dass ein Schneideffekt der Sonde nicht auftreten kann. Andererseits sind die Spannungen so hoch, dass eine isoloierende Schicht an der Sonde, die durch Verschmutzung entstehen kann, durch einen Funken durchschlagen wird. Die Koagulation mit Spannungspulsen kann somit auch durchgeführt werden, wenn nicht sichergestellt werden kann, dass die Sonde beim Koagulieren metallisch blank ist.

Eine Vorrichtung zur Minimierung der Leistung beim Schneiden menschlichen Gewebes mit Hochfrequenzstrom ist in der Deutschen Patentschrift P 25 04 280 beschrieben. Bei dieser Vorrichtung wird mit Hilfe einer Anzeigevorrichtung das Ausmaß des Lichtbogens zwischen der Schneidelektrode und dem zu schneidenden Gewebe festgestellt und das daraus abgeleitete elektrische Signal einer Regeleinrichtung zugeführt. Die Regeleinrichtung vergleicht dieses Signal mit dem Sollwertprogramm eines Sollwertgebers und leitet daraus eine Regelgröße ab, die die Ausgangsstromstärke des Generators so einstellt, dass die Intensität des Lichtbogens dem Sollwertprogramm folgt. Es hat sich aber gezeigt, dass mit konstanter Lichtbogenintensität nicht gleichzeitig ein konstantes Ergebnis der Koagulation, d.h. der Blutstillung der Schnittflächen verbunden ist. Offenbar sind die Bedingungen für gleichmäßiges Schneiden und gutes Koagulieren so verschieden, dass nicht beide Vorgänge mit einer einzigen, gemeinsamen Maßnahme, wie einer konstanten Lichtbogenintensität, gleichzeitig optimal durchgeführt werden können. Aus diesem Grund ist in der Deutschen Patentschrift P 25 04 280 ein Sollwertgeber vorgesehen, der nach einem Sollwertprogramm die Lichtbogenintensität so regelt, dass sich Zeitintervalle, in denen eine zum Schneiden erforderliche Stromstärke und Zeitintervalle, in denen eine zum Koagulieren notwendige Stromstärke des Hochfrequenzgenerators eingestellt ist, abwechseln. Zur Aufstellung eines Sollwertprogramms ist aber eine genaue Kenntnis der während einer Operation zu erwartenden Parameter, wie Gewebeart, Flüssigkeitsgehalt, Durchsetzung des Gewebes mit Blutgefäßen, Schnittgeschwindigkeit, Schnitt-Tiefe usw. nötig. In der Praxis ist daher die Aufstellung eines solchen Sollwertprogramms oft nur unvollkommen, manchmal auch gar nicht möglich. Selbst bei geringen Abweichungen der wirklichen von den angenommenen Parametern kann das Sollwertprogramm so weit vom Optimum abweichen, dass dann weder der Schneideffekt noch der Koagulationseffekt wirklich optimal sind.
Ein solches Sollwertprogramm ist auch unflexibel, wenn bei einem früher geführten Schnitt ein sehr großes Blutgefäß eröffnet wurde, das beim eingestellten Sollwertprogramm nicht vollständig verschlossen wurde. In diesem Fall muss in einem nachfolgenden Vorgang eine reine Koagulation ohne Schneidwirkung durchgeführt werden. Dies ist mit dem beschriebenen Sollwertprogramm nicht möglich, da sich hier Zeitintervalle mit Koagulationswirkung und Zeitintervalle mit Schneidwirkung gegenseitig abwechseln. Nach dem Stand der Technik ist in diesem Fall nur Koagulation mit gepulstem, ungeregeltem Koagulationsstrom möglich. Auch dabei können die Fälle mit zu hoher oder zu niedriger Generatoreinstellung auftreten. Bei zu hoher Leistungseinstellung des Hochfrequenzgenerators tritt wieder ein Lichtbogen mit allen beschriebenen Nachteilen auf, und gleichzeitig kann sich natürlich auch wieder die in diesem Moment unerwünschte Schneidwirkung einstellen. Bei zu geringer Leistungseinstellung des Hochfrequenzgenerators reicht die Koagulationswirkung nicht aus und die Blutung kann nicht oder nur ungenügend zum Stillstand gebracht werden.

Zur Lösung dieses Problems ist in der Deutschen Patentschrift DE 35 15 622 ein Generator beschrieben, der mit Hilfe von Messeinrichtungen, einer Regeleinrichtung und eines zusätzlichen Zeitgebers die Ausgangsspannung des Generators so verändert, dass sich drei Zeitabschnitte mit genau beschreibbaren Zuständen der Ausgangsspannung in laufender Folge wiederholen.

Wie Messungen im Labor gezeigt haben, ist mit diesem Generator in vielen Fällen ein ausreichendes Operationsergebnis erreichbar. Dies ist besonders dann der Fall, wenn konstante Gewebeverhältnisse vorherrschen und die Schneidelektrode mit einer konstanten Geschwindigkeit durch das Gewebe bewegt wird. Ein praktischer Einsatz eines solchen Verfahrens ist denkbar in der Urologie. Hier ist die Schneidschlinge mechanisch starr an den Betätigungshebel gekoppelt, so dass sich diese mit einer definierten, konstanten Geschwindigkeit bewegen lässt. Auch ist hier das Operationsfeld so übersichtlich und alle möglichen Operationsstellen sind gut erreichbar, so dass im Falle einer misslungenen Koagulation die blutende Stelle nachkoaguliert werden kann.

Bei anderen Operationstechniken wie die Polypektomie, bei der der Operationsort nur sehr schwer zugänglich und das Operationsfeld unübersichtlich ist, kann eine Nachkoagulation kaum mehr durchgeführt werden. Zur Abtragung von Polypen werden vorzugsweise Drahtschlingen verwendet, die über ein langes Endoskop an den Operationsort geführt werden. Wegen des mechanischen Aufbaus dieser Instrumente ist eine Bewegung der Schneidschlinge mit definierter Geschwindigkeit nicht möglich. Auch die Zugkraft lässt sich aufgrund der Reibung im Instrument nur sehr grob dosieren. Untersuchungen im Labor haben mit dem oben genannten Generator und typischen Polypektomieschlingen ein sehr schlecht reproduzierbares Koagulatuionsverhalten gezeigt. So ist bei manchen Schnitten eine durchaus gute Kaogulation erreichbar, während bei anderen Schnitten das Gewebe in kürzester Zeit ohne Koagulation glatt durchtrennt wird. Dieser Mangel tritt vor allen dann auf, wenn der Schnitt durch Bereiche mit unterschiedlichem Gewebearten führt oder wenn die Schneidelektrode eine Kruste aus Blutkoagel und verklebten Gewebeteile angesetzt hat.

Bei Koagulationen kommt es häufig vor, dass die Sonde mit der Gewebeoberfläche verklebt. Hier haften Gewebereste und koagulierte Eiweiße fest an der Sondenoberfläche und verbinden diese mit dem koagulierten Gewebe. Versucht nun der Operateur mit größerem Kraftaufwand von der Koagulationsstelle abzureißen, so löst sich häufig ein Teil der koagulierten Gewebeoberfläche mit ab. Als Folge kann die soeben koagulierte Blutung wieder beginnen. Dadurch wird eine weitere Koagulation notwendig. Außerdem erschwert eine mit Geweberesten verunreinigte Sonde weitere Koagulationen und muss vor der nächsten Koagulation gereinigt werden. Bei endoskopischen Operationstechniken wie in der Urologie ist dies mit einem hohen Aufwand verbunden, da zuvor das Operationsinstrument aus dem Körper entfernt werden muss.

Um ein Verkleben der Sonde mit dem Gewebe zu verhindern, werden Koagulatoren, die ein flüssiges oder gasförmiges Medium zur Energieübertragung verwenden eingesetzt. Diese bauen zwischen der Elektrode und dem Gewebe eine leitfähige Zwischenschicht auf. Es sind auch Geräte, die einen ionisierten Gasstrahl zur Koagulation verwenden, bekannt. Diese sind zum Beispiel beschrieben in: H.D. Reidenbach, Hochfrequenz- und Lasertechnik in der Medizin, Springer Verlag, 1983. Hierbei ist allerdings der gerätetechnische Aufwand zur Flüssigkeits- bzw. Gaseinspeisung an die Elektrodenspitze beträchtlich. Wie Untersuchungen im Labor gezeigt haben, ist mit diesen Vorrichtungen nur eine oberflächliche Koagulation möglich. Tiefenkoagulationen und moderne bipolare Koagulationstechniken sind mit diesen Vorrichtungen nicht durchführbar. Dazu sind Sonden notwendig, die unmittelbar auf der Gewebeoberfläche aufliegen. Eine Verwendung der gleichen Sonde zum Schneiden und Koagulieren ist nicht möglich. Insbesondere ist ein automatisches Umschalten in eine zweite Betriebsart Schneiden zur Entfernung der Sonde nicht möglich, so dass die Gefahr besteht, nach Beendigung des Koagulierens die koagulierte Schicht wieder abzureißen.

Aus der DE 35 30 335 A1 ist ein Hochfrequenz - Chirurgiegerät zum Schneiden und /oder Koagulieren biologischer Gewebe bekannt, das mindestens einen Hochfrequenzoszillator aufweist, der durch voneinander unabhängige Zeitgeber so gesteuert wird, dass einstellbare Zeitintervalle zum Schneiden oder Koagulieren erzeugt werden. Dem effektiven Schneide - oder Koagulationsintervall folgen jeweils einstellbare Pausenintervalle und nach deren Ablauf automatisch weitere Schneide- und Koagulationsintervalle, solange ein Tastenschalter zum Schneiden oder Koagulieren betätigt wird. Die vorliegende Erfindung geht von dem in diesem Dokument dargestellten Stand der Technik aus. Dabei liegt der Erfindung darauf aufbauend die Aufgabe zugrunde, die von der Regeleinrichtung eingestellte momentane Ausgangsgröße noch genauer und im Operationsverlauf besser angepasst als beim Stand der Technik zu steuern. Der Vorgang soll im wesentlichen automatisiert ablaufen und den Operateur von der umständlichen Bedienung der Tastenschalter weitgehend entlasten.

In der DE 36 22 337 A1 ist ein Hochfrequenzgenerator mit automatischer Leistungsregelung für die Hochfrequenzkoagulation beschrieben, der eine Lichtbogen-Anzeigevorrichtung enthält, die das evtl. Vorhandensein eines Lichtbogens zwischen der Koagulationssonde und dem zu koagulierenden Gewebe erkennt und daraus ein elektrisches Signal bildet. Mit Hilfe eines Modulators und eines Zeitgebers wird die Ausgangsleistung des Hochfrequenzgenerators so geregelt, dass sie zunächst maximal ist, solange kein Lichtbogen brennt. Nach dem Einsetzen des Lichtbogens ist sie für eine bestimmte Zeit weiterhin maximal und wird danach für eine weitere bestimmte Zeit nach Null geregelt. Dieser Zyklus von drei Zuständen wiederholt sich, solange der Generator aktiviert ist. Der in der DE 36 22 337 A1 vorgestellte Hochfrequenzgenerator eignet sich ausschließlich für die Koagulation und bietet den Vorteil, dass sich der gewünschte Koagulationseffekt sehr schnell einstellt. Die Gefahr des Gewebeschneidens durch die Koagulationssonde wird vermieden.

Damit ist jedoch auch klar, dass sich die In der DE 36 22 337 A1 beschriebene Lösung ausschließlich zum Koagulieren eignet. Die gleichzeitige Benutzung einer Schneide- und Koagulationssonde ist nicht mittels des gleichen Hochfrequenzgenerators möglich. Die gelöste Aufgabe ist somit eine andere, als die, die mit der beanspruchten Lösung zu lösen ist.

### Darstellung der Erfindung

Der Erfindung liegt daher die Aufgabe zugrunde, einen Hochfrequenzgenerator mit einer einstellbaren Ausgangsleistung für miteinander gekoppelte Betriebsarten Koagulieren und nachfolgendes Schneiden zur Entfernung der Sonde zu schaffen, der nach der Aktivierung der Betriebsart Koagulieren automatisch nach Beendigung der Koagulation in eine zweite Betriebsart Schneiden umschaltet, die es ermöglicht, die Sonde vom Gewebe zu entfernen, ohne das dabei die Gefahr besteht, die koagulierte Schicht wieder abzureißen

Diese Aufgabe wird erfindungsgemäß mit der in Patentanspruch 1 offenbarten Maßnahme gelöst. Weiterbildungen der Erfindung sind in den Unteransprüchen gekennzeichnet.

Zur Realisierung einer Hochfrequenzkoagulation, bei der gemäß der Aufgabe der Erfindung nach der Koagulation die Sonde gefahrlos vom Gewebe entfernt werden kann ohne dabei die koagulierte Schicht zu beschädigen, wird der Hochfrequenzgenerator erfindungsgemäß zeitlich nacheinander in unterschiedlichen Betriebsarten gefahren. Eine erste Betriebsart (a) besitzt koagulierende Wirkung, während eine zweite Betriebsart (b) schneidende Wirkung besitzt. Mit der Steuereinheit werden die Betriebsarten des Generators eingestellt; nach der Aktivierung des Generators zunächst die erste Betriebsart (a) mit koagulierender Wirkung und anschließend die zweite Betriebsart (b) mit schneidender Wirkung.

Zur Koagulation in der ersten Betriebsart (a) mit koagulierender Wirkung können je nach Anwendungsfall unterschiedliche Verfahren angewandt werden. Eine Koagulation in größere Gewebetiefen kann durch die Applikation von niedrigen Spannungen erreicht werden, da dadurch das Gewebe langsam bis in tiefere Zonen erwärmt werden kann. Zur Verschorfung der Gewebeoberfläche ist die Koagulation mit kurzen Spannungspulsen hoher Amplitude zweckmäßig. Durch die hohe Spannung bildet sich ein Lichtbogen zum Gewebe aus und erreicht auch Stellen, die keinen ohmschen Kontakt zur Koagulationselektrode besitzen. Durch die hohe zugeführte Energie wird die Gewebeoberfläche schnell koaguliert und damit hochohmig. Dadurch wird ein weiterer Stromfluss, weitere Energiezufuhr und eine Erwärmung tieferliegender Gewebeschichten verhindert. Um einen Schneideffekt durch den Lichtbogen zu verhindern, wird die Generatorspannung üblicherweise gepulst, so dass die mittlere Leistung so gering ist, dass ein Schneiden nicht möglich ist.

Zur Koagulation können monopolare oder bipolare Elektrodenanordnungen eingesetzt werden. Bei bestimmten Operationstechniken wie in der Urologie, wird mit dem gleichen Instrument geschnitten und koaguliert, daher ist eine monopolare Koagulation zweckmäßig. Bei offenen Operationen kann jedoch eine separate Koagulationselektrode verwendet werden. Hier ist in vielen Fällen eine bipolare Koagulation günstiger, da diese einen definierten Stromfluss und damit eine definierte Wärmeentwicklung und Koagulation zwischen den Elektroden zur Folge hat.

In der zweiten Betriebsart (b) mit schneidender Wirkung stehen entsprechend den Anforderungen unterschiedliche Verfahren zur Verfügung. Wichtig ist hierbei, dass sich der zum Schneiden notwendige Lichtbogen mit Sicherheit trotz der koagulierten Gewebeoberfläche ausbildet. Dies kann durch Anlegen einer ausreichend hohen Spannung gewährleistet werden. Ist diese Spannung allerdings zu hoch, so wird überflüssige Energie in dem Patienten eingespeist. Dies kann mit einer Vorrichtung, wie sie im Deutschen Patent 2504280 beschrieben ist, vermieden werden. Bei dieser Vorrichtung wird mit Hilfe einer Anzeigevorrichtung das Ausmaß des Lichtbogens zwischen der Schneidelektrode und dem zu schneidenden Gewebe festgestellt und das daraus abgeleitete elektrische Signal einer Regeleinrichtung zugeführt. Die Regeleinrichtung vergleicht dieses Signal mit dem Sollwertprogramm eines Sollwertgebers und leitet daraus eine Regelgröße ab, die die Ausgangsstromstärke des Generators so einstellt, dass die Intensität des Lichtbogens dem Sollwertprogramm folgt. So wird nur die zur Aufrechterhaltung des zum Schneiden notwendigen Lichtbogens benötigte Energie abgegeben.
Eine Steuereinheit schaltet den Generator nach seiner Aktivierung zunächst in eine erste Betriebsart (a) mit koagulierender Wirkung. Nach dem Ende der Koagulation, das vom Operateur bestimmt oder vom Generator automatisch festgelegt wird, schaltet die Steuereinheit den Generator kurzzeitig in die zweite Betriebsart (b) mit schneidender Wirkung. Damit werden Zellreste, die die Gewebeoberfläche mit der Elektrode verkleben, verdampft. Beim Schneiden bildet sich an der Elektrode ein Lichtbogen aus, der jeweils zu den der Elektrode am nächsten liegenden Zellen überspringt. Diese verdampfen und es bildet sich zwischen Elektrode und Gewebeoberfläche eine Dampfschicht. Damit ist die Elektrode vom Gewebe getrennt und lässt sich leicht entfernen.

Eine besonders vorteilhafte Ausführungsform besteht darin, dass ein Zeitgeber vorhanden ist, der eine voreingestellte Zeit für das Zeitintervall Schneiden vorgibt. Diese Zeit ist so bemessen, dass sie zur Ausbildung eines Lichtbogens zwischen der Elektrode und dem Gewebe ausreicht. Sie ist jedoch so kurz, dass die Elektrode nicht nennenswert in das Gewebe eindringen kann. Für die Anwendungen, bei denen immer gleichartiges Gewebe koaguliert wird, kann die notwendige Zeit experimentell ermittelt und fest eingestellt werden.

Für Anwendungen, bei denen unterschiedliche Gewebearten koaguliert werden reicht unter Umständen eine fest vorgegebene Zeit nicht aus, um in allen Fällen eine Ablösung der Schlinge vom Gewebe zu erreichen. Deshalb wird in einer weiteren Ausführungsform die Vorrichtung zum Koagulieren so erweitert, daß eine Meßeinrichtung mit Auswerteeinrichtung vorhanden ist. Diese misst während der ersten Betriebsart (a) mit koagulierender Wirkung die elektrischen Parameter am Generatorausgang und bestimmt daraus die optimale Zeitdauer für das nachfolgende Intervall mit der zweiten Betriebsart (b) mit schneidender Wirkung. Weiterhin ist ein Zeitgeber vorhanden, der die zweite Betriebsart (b) nach einer von der Meßeinrichtung mit Auswerteeinrichtung vorgegebenen Zeit beendet. Mit dem Ausgangssignal dieser Meßeinrichtung mit Auswerteeinrichtung wird der Zeitgeber entsprechend eingestellt. Zur Messung der elektrischen Parameter am Operationsort können die Ausgangsgrößen des Generators selbst oder das Signal eines Hilfsgenerators zur Messung herangezogen werden. Beispielhaft kann während der ersten Betriebsart (a) die Gewebeimpedanz ermittelt werden und für die zweite Betriebsart (b) eine Zeitdauer vorgegeben werden, die proportional zu dieser Impedanz ist. Dadurch wird für stärker verschorfte und damit hochohmigere Gewebeoberflächen eine längere Schnittdauer und damit auch ein besseres Ablösen der Sonde ermöglicht.

Eine andere vorteilhafte Ausführung besteht darin, dass eine Auswerteschaltung vorhanden ist, die den Schnittbeginn mittelbar oder unmittelbar feststellt. In der zweiten Betriebsart (b) mit schneidender Wirkung gibt diese ein Signal an die Steuereinheit ab, so daß das Zeitintervall mit der zweiten Betriebsart (b) eine voreinstellbare Zeit nach der Feststellung des Schneidens beendet wird. Um die Sonde partiell vom Gewebe zu lösen genügt eine schneidende Wirkung für kurze Zeit. Dieses Schneiden sollte allerdings noch eine geringfügige Zeit weitergeführt werden, so daß die Sonde auf ihrer ganzen Länge sicher vom Gewebe getrennt wird. Diese Zeit muß aber so kurz gewählt werden, daß die Sonde nicht wesentlich in das Gewebe eindringen kann.

Eine weitere vorteilhafte Ausführung besteht darin, dass die Auswerteschaltung einen mechanischen Sensor enthält, der ein Eindringen der Sonde in das Gewebe feststellt. So kann durch einen Wegaufnehmer der Abstand der Sonde von dem umliegenden Gewebe ermittelt werden. Hierfür eignen sich besonders optische Sensoren, die eine berührungsfreie Wegmessung ermöglichen.

Unmittelbar nach dem Beginn des Schneidens ändert sich die Impedanz zwischen der Sonde und dem Gewebe. Wie Messungen im Labor gezeigt haben, ist im allgemeinen beim Schnittbeginn ein deutlicher Anstieg dieser Impedanz meßbar. Dieser tritt besonders beim Schneiden nach einer Niederspannungskoagulation auf. Ist dagegen nach einer Hochspannungskoagulation das Gewebe sehr hochohmig, dann kann sich beim Beginn des Schneidens die Impedanz verringern. Hier überbrückt der auftretende Lichtbogen nun die hochohmige Gewebeschicht. Daher besteht eine vorteilhafte Ausführungsform darin, daß die Auswerteschaltung eine Einrichtung zur Messung und Auswertung der Impedanz enthält. Darin wird das Meßsignal mit einem Sollwert verglichen um die Impedanzänderung zu erkennen. Anstelle des Meßsignales kann auch eine Kombination des Meßsignales mit einer oder mehreren seiner Ableitungen zur Auswertung herangezogen werden.

Kennzeichnend für das Schneiden ist der dabei auftretende Lichtbogen. Daher kann der Schnittbeginn aufgrund einer Auswertung der spektralen Verteilung des Generatorausgangssignals erkannt werden. Eine solche Vorrichtung ist bereits in der noch nicht veröffentlichten deutschen Patentanmeldung P 4126607 beschrieben. Daher besteht eine vorteilhafte Ausführungsform darin, dass die Auswerteschaltung eine Einrichtung zur Auswertung der spektralen Anteile am Generatorausgang besitzt. Zur Auswertung kann das Generatorsignal selbst oder auch das Signal eines Hilfsgenerators oder eine Kombination aus beiden Signalen herangezogen werden. Die Auswertung selbst erfolgt durch einen Vergleich der Amplituden der spektralen Anteile die vom Generator bzw. dem Lichtbogen erzeugt werden.

### Kurze Beschreibung der Zeichnungen:

Die Erfindung wird nachstehend ohne Beschränkung des allgemeinen Erfindungsgedankens anhand von Ausführungsbeispielen unter Bezugnahme auf die Zeichnung exemplarisch beschrieben, auf die im übrigen bezüglich der Offenbarung aller im Text nicht näher erläuterten erfindungsgemäßen Einzelheiten ausdrücklich verwiesen wird. Es zeigen:
- Fig. 1 :: Prinzipschaltbild eines erfindungsgemäßen HochfrequenzchirurgieGenerators zum koagulierenden Schneiden,
- Fig. 2:: Beispielhafte Darstellung der Generatorausgangsspannung im Verlauf von drei Zeitintervallen,
- Fig. 3 :: Prinzipschaltbild des Hochfrequenzchirurgiegenerators zur Ausführung der Hochfrequenzkoagulation.

### Beschreibung eines Ausführungsbeispiels

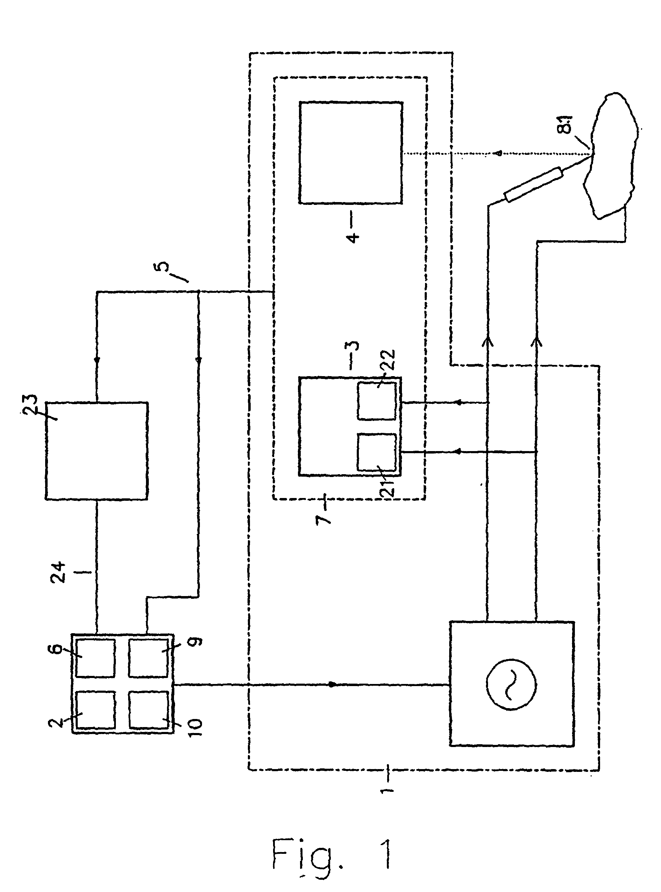

In Fig. 1 ist das Prinzipschaltbild des Hochfrequenzchirurgiegenerators nach der Erfindung dargestellt. Der Hochfrequenzgenerator (1) für die Hochfrequenzchirurgie besitzt Regeleinrichtungen zur Einstellung der momentanen elektrischen Ausgangsgrößen wie z.B. Strom, Spannung und Leistung und eine Einrichtung (7) zur unmittelbaren und oder mittelbaren Feststellung des Zustands des Gewebes in der Nähe der Schneideelektrode (8.1).

Diese Einrichtung kann einen Messgeber (4) in unmittelbarer Nähe der Schneidelektrode enthalten. Außerdem kann sie eine Auswerteeinrichtung zur Auswertung der elektrischen Ausgangsgrößen des Generators enthalten. Wahlweise kann die Einrichtung zur unmittelbaren und / oder mittelbaren Feststellung des Zustands des Gewebes in der Nähe der Schneideelektrode einen Prüfsignalgenerator (21) enthalten, dessen Prüfsignal der Schneidelektrode zugeführt und mit Hilfe der Prüfsignalmesseinrichtung gemessen wird. Weiterhin enthält die Vorrichtung einen elektronischen Speicher mit Auswerteelektronik (23), dem die Ausgangsgröße (5) der Messeinrichtung (7) zur unmittelbaren und oder mittelbaren Feststellung des Zustands des Gewebes in der Nähe der Schneideelektrode zugeführt ist. Die Ausgangsgröße (24) des elektronischen Speichers mit Auswerteelektronik und die Ausgangsgröße (5) der Messeinrichtung (7) werden dem Abschaltglied (2), dem Entscheider (6), dem Sollwertgeber (9) und dem Zeitintervallgeber (10) zugeführt.

Das Abschaltglied (2) begrenzt die Dauer des folgenden Zeitintervalles (13) anhand der Ausgangsgröße der Meßeinrichtung (7). Der Entscheider (6) wählt am Ende (15) des vorangegangenen Zeitintervalles (12) die Betriebsart für das folgende Zeitintervall (13) automatisch. Der elektronische Sollwertgeber (9) bestimmt den zeitlichen Verlauf der elektrischen Ausgangsgrößen des Hochfrequenzgenerators für die gewählte Betriebsart. Ein Zeitintervallgeber (10) legt die Dauer eines jeden Zeitintervalles aufgrund der Ausgangsgrößen des elektronischen Speichers mit Auswerteelektronik fest.

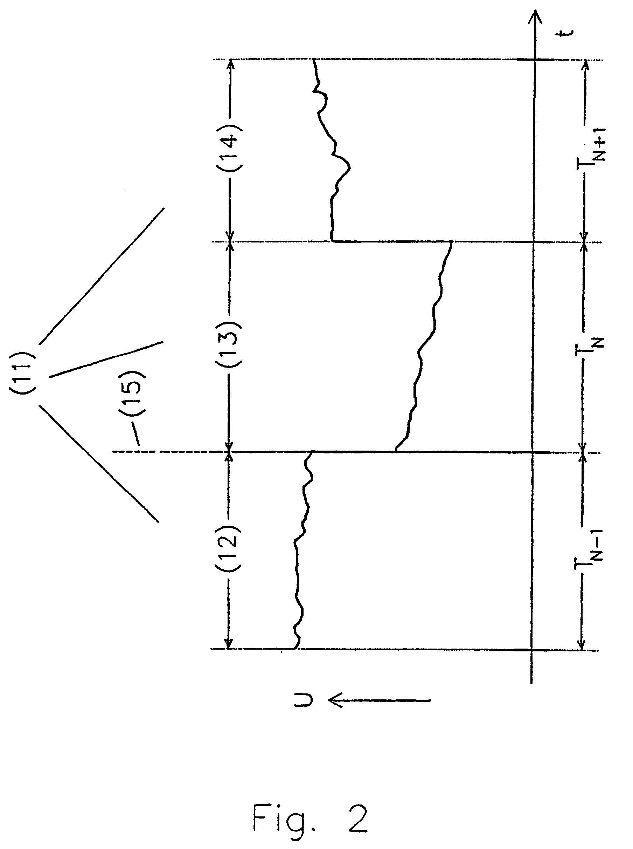

In Fig. 2 ist beispielhaft die Generatorausgangsspannung während dreier Zeitintervalle (11) dargestellt. Das folgende Zeitintervall (13) mit seiner Dauer T _{N}, auf die sich jeweils die Erklärungen beziehen, wird begrenzt durch das vorhergehende Zeitintervall (12) mit seiner Dauer T_{N-1} und das übernächste Zeitintervall (14) mit der Dauer T_{N+1}.
Der Zeitpunkt (15) befindet sich an der Grenze zwischen dem vorhergehenden und dem momentanen Zeitintervall.

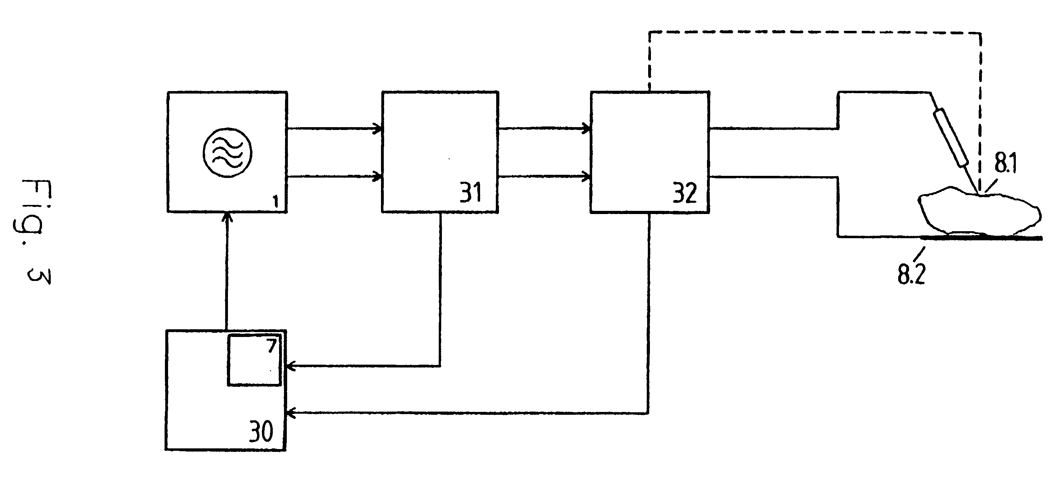

Fig. 3 zeigt das Prinzipschaltbild des Hochfrequenzchirurgiegenerators zur Ausführung der gefahrlosen Hochfrequenzkoagulation. Der Hochfrequenzgenerator (1) für die Hochfrequenzchirurgie mit einstellbarer Ausgangsleistung liefert einen hochfrequenten Strom über die Sonde (8.1) an das Gewebe. Der Strom fließt über eine weitere Elektrode (8.2) an den Generator zurück.

Die Elektrodenanordnung kann monopolar oder auch bipolar sein. Die monopolare Anordnung besteht aus einer kleinflächigen Sonde (8.1), die am Operationsort eingesetzt wird und einer großflächigen Elektrode (8.2), die an anderer Stelle am Körper des Patienten angebracht ist. Bei der bipolaren Anordnung besitzen die Sonde (8.1) und die Elektrode (8.2) gleich große Flächen und es werden beide am Operationsort mit gleicher Fläche appliziert. Mit Hilfe einer Steuereinheit (30) werden mindestens eine erste Betriebsart (a) mit koagulierender Wirkung und eine zweite Betriebsart (b) mit schneidender Wirkung realisiert. Ein Zeitgeber (10) in der Steuereinheit kann die Zeitdauer für die zweite Betriebsart (b) begrenzen. Die Messeinrichtung (31) mit Auswerteeinrichtung führt während des Zeitintervalls mit der ersten Betriebsart (a) Messungen der elektrischen Parameter am Generatorausgang durch. Mit Hilfe der Messergebnisse wird der Zeitgeber (10) für die zweite Betriebsart (b) voreingestellt. Eine Auswerteschaltung (32) dient zur mittelbaren und/oder unmittelbaren Feststellung des Schneidens. Sie gibt in der zweiten Betriebsart (b) ein Signal an die Steuereinheit (30) ab, wenn die Sonde (8.1) zu schneiden beginnt.

## Patentansprüche

1. Hochfrequenzchirurgiegenerator (1) mit einer einstellbaren Ausgangsleistung für miteinander gekoppelte Betriebsarten Koagulieren und nachfolgendes Schneiden zur Entfernung der Sonde,
**dadurch gekennzeichnet, dass** eine Steuereinheit (30) vorhanden ist, die nach der Aktivierung des Generators zunächst die erste Betriebsart (a) des Generators mit koagulierender Wirkung einstellt und automatisch am Ende der Koagulation die zweite Betriebsart (b) des Generators mit schneidender Wirkung derart kurzzeitig einstellt, dass nur die gewünschte Loslösung der Sonde vom Gewebe erfolgt, ohne dass die Sonde wesentlich in das Gewebe eindringt.

2. Hochfrequenzgenerator nach Anspruch 1,
**dadurch gekennzeichnet, dass** in der Steuereinheit (30) ein Zeitgeber (10) vorhanden ist, der am Ende des Zeitintervalls mit der ersten Betriebsart (a) des Generators mit koagulierender Wirkung für das darauffolgende Intervall mit der zweiten Betriebsart (b) des Generators mit schneidender Wirkung eine zu Beginn dieses Intervalls mit der zweiten Betriebsart (b) vorbestimmte Zeit vorgibt, die so klein ist, dass die Sonde (8.1) nicht wesentlich in das Gewebe eindringen kann.

3. Hochfrequenzgenerator nach Anspruch 1 oder 2,
**dadurch gekennzeichnet, dass** eine Messeinrichtung (31) mit Auswerteeinrichtung zur Messung der elektrischen Parameter am Generatorausgang während der ersten Betriebsart (a) des Generators mit koagulierender Wirkung vorhanden ist, die aufgrund der gemessenen Signale die optimale Zeitdauer für das folgende Zeitintervall mit der zweiten Betriebsart (b) des Generators mit schneidender Wirkung ermittelt und ein Zeitgeber (10) vorhanden ist, dem das Ausgangssignal der Messeinrichtung (31) zugeführt ist und den Zeitgeber (10) für das Zeitintervall mit der zweiten Betriebsart (b) des Generators mit schneidender Wirkung geeignet einstellt, dass nur die gewünschte Loslösung der Sonde vom Gewebe erfolgt, ohne dass die Sonde wesentlich in das Gewebe eindringt.

4. Hochfrequenzgenerator nach Anspruch 1,
**dadurch gekennzeichnet, dass** eine Auswerteschaltung (32) zur mittelbaren und/oder unmittelbaren Feststellung des Schneidens vorhanden ist, die in der zweiten Betriebsart (b) des Generators mit schneidender Wirkung ein Signal an die Steuereinheit (30) abgibt, wenn die Sonde (8.1) zu schneiden beginnt, so dass die Steuereinheit (30) das Zeitintervall mit der zweiten Betriebsart (b) eine voreinstellbare Zeit nach der Feststellung des gewebetrennenden Schneidens derart beendet, dass nur die gewünschte Loslösung der Sonde vom Gewebe erfolgt, ohne dass die Sonde wesentlich in das Gewebe eindringt.

5. Hochfrequenzgenerator nach Anspruch 4,
**dadurch gekennzeichnet, dass** die Auswerteschaltung (32) zur mittelbaren und/oder unmittelbaren Feststellung des gewebetrennenden Schneidens einen mechanischen Sensor enthält, der ein Eindringen der Sonde (8.1) in das Gewebe feststellt.

6. Hochfrequenzgenerator nach Anspruch 4,
**dadurch gekennzeichnet, dass** die Auswerteschaltung (32) zur mittelbaren und/oder unmittelbaren Feststellung des gewebetrennenden Schneidens eine Einrichtung zur Messung der Impedanz des Gewebes enthält und einen Schwellwertdetektor enthält der die beim Beginn des Schneidens typischerweise auftretende Änderung der Gewebeimpedanz erkennt.

7. Hochfrequenzgenerator nach Anspruch 4,
**dadurch gekennzeichnet, dass** die Auswerteschaltung (32) zur mittelbaren und/oder unmittelbaren Feststellung des gewebetrennenden Schneidens eine Einrichtung zur Messung der spektralen Anteile des Generatorsignals und/oder eines Hilfssignals enthält, die die typischerweise durch den beim Schneiden auftretenden Lichtbogen hervorgerufenen Harmonischen des Generatorsignals und/oder des Hilfssignals detektiert.

## Claims

1. Surgical high-frequency generator (1) with adjustable output power for mutually coupled operating modes "coagulation" and subsequent "sectioning" for removal of the probe,
**characterised in that** a controller (30) is provided that, upon activation of the generator, initially sets the first mode of operation (a) of the generator for producing a coagulating effect and automatically, by the end of coagulation, the second mode of operation (b) of the generator for a sectioning effect for such a short duration that only the desired detachment of the probe from the tissue will take place without the probe penetrating substantially into the tissue.

2. High-frequency generator according to Claim 1,
**characterised in that** a timer (10) is provided in said controller (30), which, by the end of the interval with said first mode (a) of the generator for producing a coagulating effect, predetermines a period for the following interval with said second mode (b) of the generator for producing a sectioning effect, which period is predetermined by the beginning of this interval with said second mode (b) and which is so small that said probe (8.1) cannot substantially penetrate into the tissue.

3. High-frequency generator according to Claim 1 or 2,
**characterised in that** a measuring means (31) with an evaluation system is provided for measuring the electrical parameters at the generator output during said first mode (a) of the generator for producing a coagulating effect, which means determines, on the basis of the measured signals, the optimum duration for the following interval with said second mode (b) of the generator for producing a sectioning effect, and that a timer (10) is provided which is supplied with the output signal of said measuring means (31) and which sets said timer (10) for said interval with said second mode (b) of the generator for producing a sectioning effect in such a suitable manner that only the desired detachment of the probe from the tissue will take place without a substantial penetration of said probe into the tissue.

4. High-frequency generator according to Claim 1,
**characterised in that** an evaluation circuit (32) is provided for indirect and/or direct detection of sectioning, which, during said second mode (b) of the generator for producing a sectioning effect, issues a signal to said controller (30) when said probe (8.1) begins sectioning, so that said controller (30) terminates the interval with said second mode (b) after a pre-settable period following the detection of the tissue-sectioning sectioning operation in such a way that only the desired detachment of said probe from the tissue will take place without a substantial penetration of said probe into the tissue.

5. High-frequency generator according to Claim 4,
**characterised in that** said evaluation circuit (32) comprises a mechanical sensor for indirect and/or direct detection of the tissue-sectioning operation, which sensor detects penetration of said probe (8.1) into the tissue.

6. High-frequency generator according to Claim 4,
**characterised in that** said evaluation circuit (32) for indirect and/or direct detection of the tissue-sectioning operation comprises a means for measuring the impedance in the tissue as well as a threshold detector that recognises the variation of the tissue impedance that occurs typically when the sectioning operation begins.

7. High-frequency generator according to Claim 4,
**characterised in that** said evaluation circuit (32) for indirect and/or direct detection of the tissue-sectioning operation comprises a means for measuring the spectral fractions of the generator signal and/or an auxiliary signal, which means detects the harmonics of said generator signal and/or said auxiliary signal which are typically induced by the electric arc occurring during a sectioning operation.

## Revendications

1. Générateur haute-fréquence utilisé en chirurgie (1), à rendement de sortie réglable pour des modes de service "coagulation" et ensuite "sectionnement" pour le retrait de la sonde, qui sont couplés l'un avec l'autre,
**caractérisé en ce qu'**une unité de commande (30) est disposée, qui, après la mise en circuit du générateur, commute d'abord au premier mode d'opération (a) du générateur afin de créer un effet de coagulation, et, de façon automatisée, à la fin de la coagulation, au deuxième mode d'opération (b) du générateur afin de créer un effet de sectionnement pour une durée si courte que seulement le détachement souhaité de la sonde du tissu soit achevé, sans pénétration essentielle de la sonde dans le tissu.

2. Générateur haute-fréquence selon la revendication 1,
**caractérisé en ce qu'**une minuterie (10) est disposé dans ladite unité de commande (30), qui, à la fin de l'intervalle audit premier mode (a) du générateur à produire un effet de coagulation, détermine une période pour l'intervalle suivant audit deuxième mode (b) du générateur à produire un effet de sectionnement, laquelle période est déterminée au début de cet intervalle audit deuxième mode (b) et si petit que ladite sonde (8.1) ne puisse pénétrer essentiellement dans le tissu.

3. Générateur haute-fréquence selon la revendication 1 ou 2,
**caractérisé en ce qu'**un moyen de mesure (31) à moyen analyseur est disposé à mesurer les paramètres électriques à la sortie du générateur pendant ledit premier mode (a) du générateur afin de produire un effet de coagulation, ce moyen déterminant, à la base des signaux mesurés, la durée optimale pour l'intervalle suivant audit deuxième mode (b) du générateur afin de produire un effet de sectionnement, et **en ce qu'**une minuterie (10) est comprise, à laquelle est fourni le signal de sortie dudit moyen de mesure (31) qui règle ladite minuterie (10) pour ledit intervalle audit deuxième mode (b) du générateur afin de produire un effet de sectionnement d'une manière convenable, que seulement de détachement souhaité de la sonde du tissu soit achevé sans pénétration essentielle de ladite sonde dans le tissu.

4. Générateur haute-fréquence selon la revendication 1,
**caractérisé en ce qu'**un circuit analyseur (32) est compris pour la détection indirecte et/ou directe du sectionnement, qui, au cours dudit deuxième mode (b) du générateur afin de produire un effet de sectionnement, fournit un signal à ladite unité de commande (30) dès que ladite sonde (8.1) commence le sectionnement, de façon que ladite unité de commande (30) termine l'intervalle audit deuxième mode (b) après une période pré-réglable suivant la détection de l'opération de sectionnement à couper le tissu, d'une manière que seulement de détachement souhaité de la sonde du tissu soit achevé sans pénétration essentielle de ladite sonde dans le tissu.

5. Générateur haute-fréquence selon la revendication 4,
**caractérisé en ce que** ledit circuit analyseur (32) comprend un détecteur mécanique pour la détection indirecte et/ou directe de l'opération de sectionnement, ce détecteur détectant la pénétration de ladite sonde (8.1) dans le tissu.

6. Générateur haute-fréquence selon la revendication 4,
**caractérisé en ce que** ledit circuit analyseur (32) pour la détection indirecte et/ou directe de l'opération de sectionnement à couper le tissu, comprend un moyen à mesurer l'impédance dans le tissu ainsi qu'un détecteur de seuil, qui détecte la variation de l'impédance dans le tissu, qui est présente, au cas typique, au début de l'opération de sectionnement.

7. Générateur haute-fréquence selon la revendication 4,
**caractérisé en ce que** ledit circuit analyseur (32) pour la détection indirecte et/ou directe de l'opération de sectionnement à couper le tissu comprend un moyen à mesurer les fractions spectrales du signal du générateur et/ou d'un signal auxiliaire, ce moyen détectant des harmoniques dudit signal du générateur et/ou dudit signal auxiliaire, qui sont induits, au cas typique, par l'arc électrique établi au cours d'une opération de sectionnement.
